# EUROPÄISCHE PATENTSCHRIFT

(11) **EP 2 193 837 B1**
(45) Veröffentlichungstag und Bekanntmachung des Hinweises auf die Patenterteilung: **03.10.2012**
(21) Anmeldenummer: 09178130.2
(22) Anmeldetag: 06.12.2009
(51) Int. Cl.: B01F 7/06, A01C 3/02

(54) **Rührwerk**
Stirring assembly
Agitateur

(30) Priorität: 07.12.2008 DE 202008015990 U
(43) Veröffentlichungstag der Anmeldung: 09.06.2010
(73) Patentinhaber: Thürwächter GmbH & Co.KG, 87477 Sulzberg (DE)
(72) Erfinder: Thürwächter, Paul, 87477, Sulzberg (DE)
(74) Vertreter: Hutzelmann, Gerhard

(56) Entgegenhaltungen:
- EP-A1- 1 992 405
- EP-A2- 1 541 224
- DE-A1- 2 938 177

## Beschreibung

Die Erfindung bezieht sich auf ein Rührwerk entsprechend Anspruch 1, insbesondere Güllerührwerk, mit einem Antriebsmotor und einer Rührwerksschraube, das an einer Wand eines Behälters angeordnet ist, wobei die Rührwerksschraube in einem Rohr längsverschiebbar gelagert ist und wobei die Antriebswelle mit einem festen und einem längs verschiebbaren Lager versehen ist.

Aus der EP 1 992 405 ist beispielsweise ein Rührwerk entsprechend dem Oberbegriff des Anspruch 1 bekannt.

Der Erfindung liegt die Aufgabe zugrunde, ein Rührwerk der genannten Art zu schaffen, das auf unterschiedliche Betriebsbedingungen einstellbar ist bei größerer Stabilität der Führung der Antriebswelle.

Diese Aufgabe wird erfindungsgemäß dadurch gelöst, dass das längsverchiebbare Lager im Rohr angeordnet ist und einerseits auf der Antriebswelle verschiebbar ist und zugleich im Rohr verschoben werden kann.

Dadurch kann die Position der Rührwerksschraube leicht in ihrer Lage verändert werden.

Erfindungsgemäß ist die Rührwerksschraube in einem Rohr längsvorschiebbar gelagert.

Dabei kann die Rührwerksschraube vollständig in das Rohr einziehbar gelagert sein.

Damit ist bei Nichtbedarf der Rührwerksschraube deren weitgehender Schutz gewährleistet.

Eine weitere vorteilhafte Ausgastaltung der Erfindung ist **dadurch** gekennzeichnet, **dass** am offenen Ende des Rohres ein Verschlusselement vorgesehen ist.

Damit kann im eingezogenen Zustand der Rührwerksschraube das Rohr verschlossen werden, womit die Rührwerksschraube einen sehr guten Schutz erhält.

Dies ist vorallem dann gewährleistet, wenn erfindungsgemäß der Verstellweg der Rührwerksschraube so gestaltet ist, dass die Rührwerksschraube hinter das Verschlusselement eingezogen werden kann.

Als sehr vorteilhaft hat es sich dabei ergeben, wenn gemäß einer Ausgestaltung der Erfindung das Verschlusselement als Rohrschieber ausgebildet ist.

Derartige Verschlusselemente sind soweit dicht, dass die Rührwerksschraube auch zu Wartungsarbeiten herausgenommen werden kann.

Gemäß einer weiteren Ausgestaltung der Erfindung ist es jedoch auch möglich, dass die Antriebswelle längenveränderlich ausgebildet ist.

Als ebenfalls sehr vorteilhaft hat es sich ergeben, wenn gemäß einer weiteren Ausgestaltung der Erfindung das Rohr im wesentlichen ausserhalb des Behälters angeordnet ist.

Damit ist bei eingezogener Rührwerksschraube im Inneren des Güllebehälters kein störendes Bauteil mehr vorhanden.

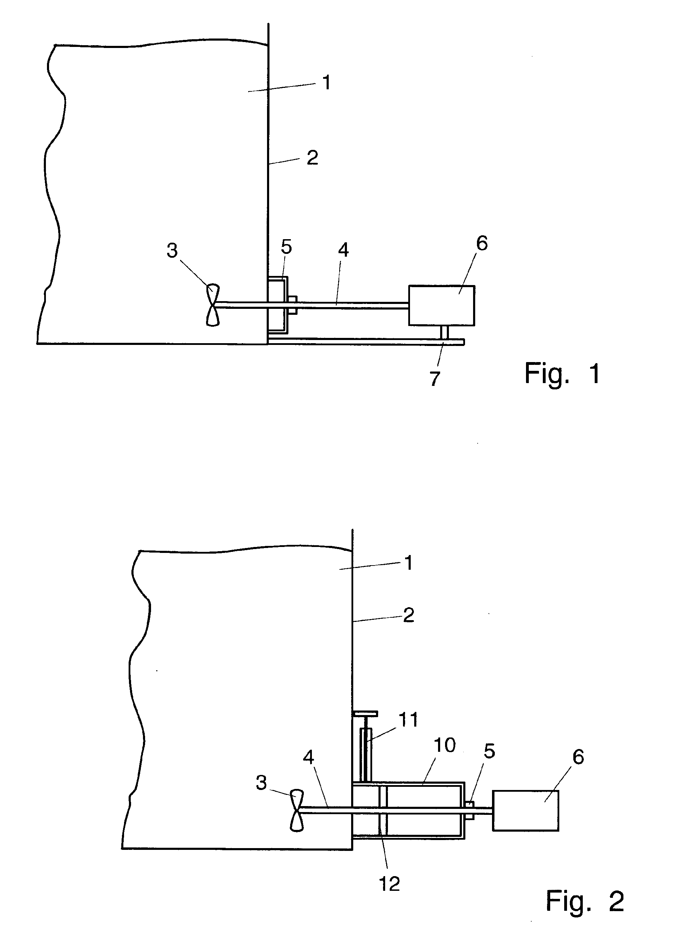

In der Zeichnung sind zwei Ausführungsbeispiele veranschaulicht, wobei das Ausführungsbeispiel der Fig. 1 nicht Teil der Erfindung ist. Dabei zeigen:
Fig. eine schematische Darstellung einer Rührwerksschraube, die in einer Wand eines Güllebehälters längsverschiebbar gelagert ist und
Fig.2 eine ebenfalls schematische Darstellung einer Rührwerksschraube, die in einem Rohr längsverschiebbar gelagert ist, wobei das Rohr von aussen in die Wand des Güllebehälters hineinragt.

Mit 1 ist in Fig.1 ein Güllebehälter bezeichnet, in dessen vertikaler Wand 2 in Bodennähe eine Rührwerksschraube 3 im wesentlichen horizontal gelagert ist. Die Rührwerksschraube 3 sitzt auf einer Antriebswelle 4, die durch die Wand 2 hindurchgreift und in einem Lager 5 an der Wand 2 gelagert ist. Am von der Rührwerksschraube abgekehrten Ende der Antriebswelle 4 ist ein Antriebsmotor 6 angeordnet, der auf einem Lager 7 längsverschiebbar gelagert ist. Damit kann die gesamte Einheit aus Rührwerksschraube, Antriebswelle und Antriebsmotor in Längsrichtung der Antriebswelle verschoben werden.

Anstelle der längsverschiebbaren Lagerung des Antriebsmotors ist es auch möglich, die Antriebswelle insich längenveränderlich auszubilden.

Bei beiden Ausgestaltungen kann das Lager 5 in Durchmesser so groß ausgebildet werden, dass die Rührwerksschraube 3 in dieses Lager eingezogen werden kann.

Beim Ausfübrungsbeispiel nach Fig.2 ist in die Wand 2 des Güllebehälters 1 ein Rohr 10 von außen eingesetzt, durch welches die Antriebswelle 4 hindurchragt. Der Durchmesser des Rohres 10 ist so gewählt, dass die Rührwerksschraube 3 beim Zurückziehen in das Rohr 10 einzufahren vermag.

In der Nähe der Wand 2 ist am Rohr ein Rohrschieber 11 angeordnet, welcher bei eingefahrener Rührwerksschraube das Rohr anzuschließen vermag.

Dadurch ist zum einen die Rührwerksschraube geschützt und kann zu Wartungsarbeiten herausgenommen werden auch wenn der Güllebehälter 1 gefüllt ist.

Um eine größere Stabilität der Führung der Antriebswelle 4 zu erreichen ist ein Stützlager 12 vorgesehen, welches einerseits auf der Antriebswelle 4 verschiebbar ist und zugleich im Rohr 10 verschoben werden kann.

## Patentansprüche

1. Rührwerk, insbesondere Güllerührwerk, mit einem Antriebsmotor (6) und einer am Ende einer Antriebswelle (4) angeordneten Rührwerksschraube (3), wobei das Rührwerk an einer Wand eines Behälters (1) angeordnet ist, wobei die Rührwerksschraube (3) in einem Rohr (10) längsverschiebbar gelagert ist und wobei die Antriebswelle (4) mit einem festen (5), und einem längsverschiebbaren Lager (12) versehen ist, **dadurch gekennzeichnet, daß** das längsverschuebbare Lager (12) im Rohr (10) angeordnet ist und einerseits auf der Antriebswelle (4) verschiebbar ist und zugleich im Rohr (10) verschoben werden kann.

2. Rührwerk nach Anspruch 1, **dadurch gekennzeichnet, dass** die Rührwerksschraub (3) vollständig in das Rohr (10) einziehbar gelagert ist.

3. Rührwerk nach Anspruch 2, **dadurch gekennzeichnet, dass** am offenen Ende des Rohres (10) ein Verschlusselement (11) vorgesehen ist.

4. Rührwerk nach Anspruch 3, **dadurch gekennzeichnet, dass** der Verstellweg der Rührwerksschraube (3) so gestalltet ist, dass die Rühwerksschraube (3) hinter das Verschlusselement (11) eingezogen werden kann.

5. Rührwerk nach einem der Ansprüche 3 oder 4, **dadurch gekennzeichnet, dass** das Verschlusselement als Rohrschieber (11) ausgebildet ist.

6. Rührwerk nach einem der vorhergehenden Ansprüche, dadurch gekenntzeichnet dass die Antriebswelle (4) längenveränderlich ausgebildet ist.

7. Rührwerk nach einem der Ansprüche 1 bis 6, **dadurch gekennzeichnet, dass** das Rohr (10) im wesentlichen ausserhalb des Behälters (1) angeordnet ist.

## Claims

1. Stirring mechanism, particularly manure stirring mechanism, with a drive motor (6) and a stirring mechanism screw (3) arranged at the end of a drive shaft (4), wherein the stirring mechanism is arranged at a wall of a container (1), wherein the stirring mechanism screw (3) is mounted in a tube (10) to be longitudinally displaceable and wherein the drive shaft (4) is provided a fixed bearing (5) and a longitudinally displaceable bearing (12), **characterised in that** the longitudinally displaceable bearing (12) is arranged in the tube (10) and on the one hand is displaceable on the drive shaft (4) and at the same time can be displaced in the tube (10).

2. Stirring mechanism according to claim 1, **characterised in that** the stirring mechanism screw (3) is mounted to be completely retractable into the tube (10).

3. Stirring mechanism according to claim 2, **characterised in that** a locking element (11) is provided at the open end of the tube (10).

4. Stirring mechanism according to claim 3, **characterised in that** the adjustment path of the stirring mechanism screw (3) is so designed that the stirring mechanism screw (3) can be withdrawn behind the locking element (11).

5. Stirring mechanism according to one of claims 3 and 4, **characterised in that** the locking element is constructed as a sleeve valve (11).

6. Stirring mechanism according to any one of the preceding claims, **characterised in that** the drive shaft (4) is constructed to be variable in length.

7. Stirring mechanism according to any one of claims 1 to 6, **characterised in that** the tube (10) is arranged substantially outside the container (1).

## Revendications

1. Agitateur, notamment agitateur à lisier comprenant un moteur d'entraînement (6) et une vis d'agitation (3) disposée à l'extrémité d'un arbre d'entraînement (4), ledit agitateur étant implanté sur une paroi d'un conteneur (1), ladite vis d'agitation (3) étant montée à coulissement longitudinal dans une tubulure (10), et ledit arbre d'entraînement (4) étant pourvu de paliers fixe (5) et apte au coulissement longitudinal (12), **caractérisé par le fait que** le palier (12) apte au coulissement longitudinal est logé dans la tubulure (10) et peut, d'une part, coulisser sur l'arbre d'entraînement (4) en pouvant, dans le même temps, être animé de coulissements dans ladite tubulure (10).

2. Agitateur selon la revendication 1, **caractérisé par le fait que** la vis d'agitation (3) est montée avec faculté de rétraction intégrale dans la tubulure (10).

3. Agitateur selon la revendication 2, **caractérisé par le fait qu'**un élément d'obturation (11) est prévu à l'extrémité ouverte de la tubulure (10).

4. Agitateur selon la revendication 3, **caractérisé par le fait que** le trajet de réglage de la vis d'agitation (3) est conçu de façon telle que ladite vis d'agitation (3) puisse être rétractée derrière l'élément d'obturation (11).

5. Agitateur selon l'une des revendications 3 ou 4, **caractérisé par le fait que** l'élément d'obturation est réalisé sous la forme d'un tiroir tubulaire (11).

6. Agitateur selon l'une des revendications précédentes, **caractérisé par le fait que** l'arbre d'entraînement (4) est réalisé avec longueur variable.

7. Agitateur selon l'une des revendications 1 à 6, **caractérisé par le fait que** la tubulure (10) se trouve, pour l'essentiel, à l'extérieur du conteneur (1).
